# EUROPEAN PATENT APPLICATION

(11) **EP 2 174 650 A1**
(43) Date of publication of application: **14.04.2010**
(21) Application number: 08166058.1
(22) Date of filing: 08.10.2008
(51) Int. Cl.: A61K 9/00, A61K 9/06, A61K 9/107, A61K 31/345, A61K 31/4164, A61K 31/4174, A61K 31/4412, A61K 31/4418, A61K 31/4745, A61K 31/522, A61K 31/569, A61P 15/02, A61P 15/12, A61P 17/00

(54) **Modified release emulsions for application to skin or vaginal mucosa**

(71) Applicant: Polichem SA, 1526 Luxembourg (LU)
(72) Inventor: Mura, Emanuela, I-22100, COMO (IT); Mailland, Federico, I-22100, COMO (IT); Ceriani, Daniela, I-21050, Besano (Varese) (IT)
(74) Representative: Pistolesi, Roberto

(57) **Abstract**

Modified release oil in water emulsions that delivers drugs to a target tissue. The emulsions according to the present invention contain the lipophilic active ingredient completely dissolved into the hydrophobic internal phase stabilised by a polymeric surfactant. The presence of this polymer around the hydrophobic droplets avoids the migration of the active ingredient into the external hydrophilic phase and, consequently, its re-crystallization.

## Description

This invention relates to oil in water emulsions, suitable to be applied on skin or mucosal surfaces, containing a water insoluble active principle completely dissolved into the internal hydrophobic phase stabilized by a polymeric surfactant from the external hydrophilic phase.

The active principle trapped and stabilized into the internal hydrophobic phase does not migrate into the hydrophilic phase and consequently does not re-crystallize even stored at 40°C for 6 months.

### BACKGROUND OF THE INVENTION

Water insoluble active principles are difficult to be formulated. In the art, it is known that a drug can be dissolved by using a similar solvent. Consequently, a lipophilic drug is solubilized by using an apolar solvent, e.g. an oil.

To be applied on the skin or mucosal surfaces, oil in water or water in oil compositions, in form of oil in water or water in oil creams and lotions, are an attractive way for drugs formulation.

Semisolid emulsions (i.e. creams and lotions) are two-phases compositions in which one phase (the dispersed or internal phase) is finely dispersed in the other (the continuous or external phase). The dispersed phase can be either hydrophobic based (oil in water creams) or aqueous based (water in oil creams). It is known that a cream is oil in water or water in oil depending on the properties of the system used to stabilize the interface between the phases.

It is known that the use of the appropriate surfactant (i.e. surface active ingredient) improves the physical stability of an emulsion, decreasing the contact angle between the apolar and polar surfaces and, consequently, the active ingredient dissolved into a phase. In most pharmaceutical emulsions stabilizing systems are comprised of either ionic and non ionic surfactants. However, surfactant molecules tend to self-associate, forming micellar or lamellar structures, modifying the stability of the emulsion. Over the time, semisolids tend to modify their physical-chemical properties (i.e. viscosity, appearance and homogeneity) and the active ingredient tends to re-crystallize, due to its migration to the other phase modifying its performances, such as homogeneous distribution into the final product and its delivery.

### DESCRIPTION OF THE INVENTION

The O/W emulsions object of this invention are stabilized by using a polymeric surfactant, which avoid modification of the internal phase.

Use of polymeric surfactants is already known in the art. Stabilizing systems comprise non-ionic polymers e.g. poloxamer block copolymers) or polyelectrolites (e.g polyacrylic/polymethacrylic acids) or mixture of these. Emulsions made by using these molecules are more stable.

Polymers of acrylic acid, such as Pemulen® TR-1 and 2 can be used at very low concentrations (0.2-0.5%(w/w), jellifying around the droplets of the dispersed hydrophobic phase. Generally, these kind of polymers have to be "activated" by using, e.g. sodium hydroxide, potassium hydroxide, ammonium hydroxide, organic amine bases such as triethanolamine ,tromethamine, aminomethyl propanol. These polymer activactors have to be already into the water phase before the emulsification step. The activation converts the coiled form to the uncoiled one of the polymers, which organize around the droplet.

In this invention, the use of specific polymeric surfactants at specific concentrations not only stabilizes the physical characteristics of the final product (i.e. phases do not separate or change their viscosity), but, surprisingly, the migration of the active ingredient into the external hydrophilic phase is avoided. Consequently, it does not re-crystallize, even when stored for 6 months at 40°C.

The O/W emulsions according to the present invention are preferably in form of lotions, creams or gels, and are preferably topically applied to the skin or into the vaginal cavity by a suitable applicator.

These emulsions contain an internal hydrophobic phase in amounts ranging between 1 to 40% by weight, preferably from 5 to 30%, more preferably from 10 to 25%,with respect to the weight of the emulsion; said internal hydrophobic phase preferably contains benzyl alcohol and 2-octyldodecanol, more preferably in a ratio ranging from 1:3 to 1:13 by weight, preferably from 1:5 to 1:11, wherein preferred ratios are 1:5, 1:10 or 1:11; the internal hydrophobic phase may also contain other hydrophobic excipients, which are preferably selected from the group comprising medium-chain mono-, di- and triglycerides (i.e. from 6 to 12 carbon atoms mono-, di- and tri- fatty acid esters of glycerol), polyethylene glycol, isopropyl myristate, mineral oils, silicone oils, vegetable oils, such as coconut, cotton seed, peanut, olive, palm, sunflower seed, sesame, corn, soybean oil, or a mixture combination thereof.

The emulsions contain an external hydrophilic phase in amounts ranging between 60 to 99% by weight, preferably from 70 to 95%, more preferably from 75 to 90 %, with respect to the weight of the emulsion; said hydrophilic phase preferably contains lower alkanols, polyhydric alcohols, polyethylene glycols, polypropylene glycols or mixtures thereof and not more than 60% by weight of purified water; the weight percentage is intended with respect to the weight of the hydrophilic phase.

The present O/W emulsions contain at least one polymeric surfactant in amounts ranging from 0.50 to 2.50% by weight, preferably from 1.00 to.2.00%, with respect to the weight of the emulsion; said polymeric surfactant is preferably selected from the group of acrylates/C₁₀-C₃₀ alkyl acrilate crosspolymers (i.e. high molecular weight copolymer of acrylic acid and a long chain alkyl methacrylate crosslinked with allyl ethers of pentaerythritol) or from the group of cellulose ethers, such as alkylcellulose, preferably methylcellulose, and hydroxyalkylcellulose hydroxypropylmethylcellulose (preferably Methocel^{®} A and K types). Both types of celluloses have a backbone of cellulose but different ratios of hydroxypropyl to methoxyl substitution.

These concentrations are important to obtain a thick layer around the droplet like a "wall" to trap the lipophylic active ingredient. Addition of neutralising agents is not necessary to stabilise the system.

The emulsions may contain a bio/mucoadhesive ingredient in a proportion ranging between 0.5 to 1.5%, with respect to the weight of the emulsion; said bio/mucoadhesive ingredient being selected from the group of Carbomers, dispersed into the hydrophilic phase.

The emulsions also may contain jellifying agents, selected from the groups of semisynthetic celluloses, comprising methylcellulose, carboxymethyl cellulose, hydroxyethyl cellulose, hydroxypropylcellulose, hydroxypropylmethyl cellulose, propylcellulose, polysaccarides gums, such as tragacanth, pectin, carrageenan and guar, alginic acid and its sodium salt and Poloxamers.

The emulsions contain at least a water insoluble active pharmaceutical principle, dissolved into the internal hydrophobic phase, in amounts ranging from 0.01 to 25 % by weight, preferably from 0.5 to 15%, more preferably from 1.0 to 10%, with respect to the weight of the emulsion. The active principle is preferably useful in specific and non specific infections (due to e.g. bacteria, fungi and protozoa) or anti-inflammatory drugs. Compositions may also be useful to deliver hormones. The compositions will be prepared according to conventional techniques, and may include compatible excipients and pharmaceutically acceptable carriers, e.g. ionizing agents, antioxidant agents, chelating agents, moisturizing agents, decongestant agents, preservatives, disinfectant and/or antimicrobial agents, flavoring and colorants.

The compositions may also contain, in combination, other active principles with complementary or, in any case, useful activity. Examples of these compositions prepared according to the present invention include:
lotion, cream or jellified emulsion.

The pharmaceutical compositions and the uses of the present invention will be described in details by the following examples. It should, however, be noted that such examples are given by way of illustration and not of limitation.

### EXAMPLE 1

A jellified oil in water emulsion having the following w/w % composition was prepared:

| | | |
|---|---|---|
| 1) | Ciclopirox USP | 0.77% |
| 2) | Polycarbophil¹ | 1.00% |
| 3) | Glycerol USP | 15.00% |
| 4) | Peanut Oil | 9.00% |
| 5) | Acrylates/C10-C30 Alkyl Acrylates² | 1.00% |
| 6) | PEG-8 Caprylic/Capric Glyceride³ | 2.00% |
| 7) | Propylene Glycol USP | 25.00% |
| 8) | 2-Octyl Dodecanol⁴ | 11.00% |
| 9) | Benzyl Alcohol | 1.00% |
| 10) | Purified Water | 34.23% |

| | | |
|---|---|---|
| ¹Noveon AA1^{®}; ²Pemulen TR-1; ³Labrasol; ⁴Eutanol G | | |

Cyclopirox was dissolved by magnetic stirring with Benzyl Alcohol and Octyl dodecanol at room temperature. Then, Peanut Oil, Labrasol and Pemulen TR-1 were added and mixed to obtain a homogeneous suspension (Phase A). Glycerol, Propylene Glycol and water were mixed at room temperature. (Phase B). Phase A was added to Phase B at room temperature and mixed at elevated rpm until a homogeneous semisolid formulation was obtained. Polycarbophil was added and the formulation was gently mixed until a homogeneous jellified emulsion was obtained. The obtained jellified emulsion was white and homogeneous in appearance. A light microscopy analysis did not show presence of active ingredient's crystals.

### EXAMPLE 2

An oil in water lotion formulation having the following w/w % composition was prepared:

| | | |
|---|---|---|
| 1) | Ciclopirox USP | 0.77% |
| 2) | Glycerol USP | 15.00% |
| 3) | Peanut Oil | 9.00% |
| 4) | PEG-6 stearate(and) PEG-32 stearate¹ | 5.00% |
| 5) | Acrylates/C10-C30 Alkyl Acrylates² | 2.00% |
| 6) | Propylene Glycol USP | 15.00% |
| 7) | Mineral Oil | 2.00% |
| 8) | 2-Octyl Dodecanol³ | 9.00% |
| 9) | Benzyl Alcohol | 1.00% |
| 10) | Purified Water | 40.23% |

| | | |
|---|---|---|
| ¹tefose 1500^{®}; ²Pemulen^{®} TR-1; ³Eutanol G | | |

Cyclopirox was dissolved by magnetic stirring with Benzyl Alcohol and Octyl dodecanol at room temperature. Peanut Oil, Mineral oil and Pemulen TR-1 were added and mixed. Tefose 1500 was melted, added to the previous ingredients and mixed until a homogeneous suspension was obtained (Phase A). Glycerol, Propylene Glycol and water were mixed and lightly heated. (Phase B). Phase A was added to Phase B and mixed at elevated rpm decreasing the temperature until a homogeneous semisolid formulation was obtained. The obtained lotion was white, homogeneous in appearance with a viscosity around 1500mPas. A light microscopy analysis did not show presence of active ingredient's crystals.

### EXAMPLE 3

An oil in water cream formulation having the following w/w % composition was prepared:

| | | |
|---|---|---|
| 1) | Nifuratel | 10.00% |
| 2) | Glycerol USP | 15.00% |
| 3) | Almond Oil | 3.00% |
| 4) | PEG-6 stearate(and)glycol stearate(and)PEG-32 stearate¹ | 8.00% |
| 5) | Hydroxypropylmethyl cellulose | 2.00% |
| 6) | Propylene Glycol USP | 14.00% |
| 7) | Mineral Oil | 2.00% |
| 8) | 2-Octyl Dodecanol³ | 9.00% |
| 9) | Benzyl Alcohol | 1.00% |
| 10) | Purified Water | 37.00% |

| | | |
|---|---|---|
| ¹Tefose^{®}63; ²Methocel^{®} K100; ³Eutanol G | | |

Nifuratel was dissolved by magnetic stirring with Benzyl Alcohol and Octyl dodecanol at room temperature. Peanut Oil, Mineral oil were added and mixed. Tefose 1500 was melted, added to the previous ingredients and mixed until a homogeneous suspension was obtained (Phase A). Glycerol, Propylene Glycol and water were mixed and lightly heated.Methocel K100 was then dissolved (Phase B). Phase A was added to Phase B and mixed at elevated rpm decreasing the temperature until a homogeneous semisolid formulation was obtained.

The obtained oil in water cream was yellow in colour, due to the presence of Nifuratel, homogeneous in appearance with a viscosity around 1500mPas. A light microscopy analysis did not show presence of active ingredient's crystals.

### EXAMPLE 4

A thermosetting gel having the following w/w % composition was prepared:

| | | |
|---|---|---|
| 1) | Ciclopirox USP | 0.77% |
| 2) | PEG 400¹ | 10.00% |
| 3) | Poloxamer 407² | 18.00% |
| 4) | Polycarbophil³ | 1.00% |
| 5) | Hydroxypropylmethyl cellulose⁴ | 1.00% |
| 6) | Propylene Glycol USP | 20.00% |
| 7) | Mineral Oil | 2.00% |
| 8) | 2-Octyl Dodecanol⁵ | 5.00% |
| 9) | Benzyl Alcohol | 1.00% |
| 10) | Purified Water | 41.23% |

| | | |
|---|---|---|
| ¹Lutrol E400; ²Lutrol F127; ³Noveon AA1^{®}; ⁴Methocel^{®} K100; ⁵Eutanol G | | |

The formulation was prepared by using the same method described for Example 4. The obtained gel was white and homogeneous in appearance. A light microscopy analysis did not show presence of active ingredient's crystals.

### EXAMPLE 5

A thermosetting gel having the following w/w % composition was prepared:

| | | |
|---|---|---|
| 1) | Ciclopirox USP | 0.77% |
| 2) | Poloxamer 407¹ | 18.00% |
| 3) | Polycarbophil² | 1.00% |
| 4) | Acrylates/C10-C30 Alkyl Acrylates³ | 1.00% |
| 5) | Propylene Glycol USP | 20.00% |
| 6) | Isopropyl Myristate⁴ | 5.00% |
| 7) | 2-Octyl Dodecanol⁵ | 10.00% |
| 8) | Benzyl Alcohol | 1.00% |
| 9) | Purified Water | 43.23% |

| | | |
|---|---|---|
| ¹Lutrol F127; ²Noveon AA1^{®}; ³Pemulen TR-1; ⁴Crodamol IPM; ⁵Eutanol G | | |

The formulation was prepared by using the same method described for Example 1. The obtained gel was white and homogeneous in appearance. A light microscopy analysis did not show presence of active ingredient's crystals.

### EXAMPLE 6

A jellified oil in water emulsion having the following w/w % composition was prepared:

| | | |
|---|---|---|
| 1) | Estradiol | 0.03% |
| 2) | Polycarbophil¹ | 1.00% |
| 3) | Glycerol USP | 15.00% |
| 4) | Peanut Oil | 9.00% |
| 5) | Acrylates/C10-C30 Alkyl Acrylates² | 1.00% |
| 6) | PEG-8 Caprylic/Capric Glyceride³ | 2.00% |
| 7) | Propylene Glycol USP | 25.00% |
| 8) | 2-Octyl Dodecanol⁴ | 11.00% |
| 9) | Benzyl Alcohol | 1.00% |
| 10) | Purified Water | 34.97% |

| | | |
|---|---|---|
| ¹Noveon AA1^{®}; ²Pemulen TR-1; ³Labrasol; ⁴Eutanol G | | |

The formulation was prepared by using the same method described for Example 1. The obtained jellified emulsion was white and homogeneous in appearance. A light microscopy analysis did not show presence of active ingredient's crystals.

### EXAMPLE 7

A thermosetting gel having having the following w/w % composition was prepared:

| | | |
|---|---|---|
| 1) | Imiquimod | 5.00% |
| 2) | Poloxamer 407¹ | 18.00% |
| 3) | Polycarbophil² | 1.00% |
| 4) | Acrylates/C10-C30 Alkyl Acrylates³ | 1.00% |
| 5) | Propylene Glycol USP | 25.00% |
| 6) | 2-Octyl Dodecanol⁴ | 10.00% |
| 7) | Benzyl Alcohol | 1.00% |
| 8) | Purified Water | 39.00% |

| | | |
|---|---|---|
| ¹Lutrol F127; ²Noveon AA1^{®}; ³Pemulen TR-1; ⁴Eutanol G | | |

The formulation was prepared by using the same method described for Example 1. The obtain gel was white and homogeneous in appearance. A light microscopy analysis did not show presence of active ingredient's crystals.

### EXAMPLE 8

A jellified oil in water emulsion having the following w/w % composition was prepared:

| | | |
|---|---|---|
| 1) | Acyclovir | 5.00% |
| 2) | Polycarbophil¹ | 1.00% |
| 3) | Glycerol USP | 15.00% |
| 4) | Peanut Oil | 9.00% |
| 5) | Acrylates/C10-C30 Alkyl Acrylates² | 1.00% |
| 6) | PEG-8 Caprylic/Capric Glyceride³ | 2.00% |
| 7) | Propylene Glycol USP | 20.00% |
| 8) | 2-Octyl Dodecanol⁴ | 11.00% |

| | | |
|---|---|---|
| 9) | Benzyl Alcohol | 1.00% |
| 10) | Purified Water | 34.00% |

| | | |
|---|---|---|
| ¹Noveon AA1^{®}; ²Pemulen TR-1; ³Labrasol; ⁴Eutanol G | | |

The formulation was prepared by using the same method described for Example 1. The obtained jellified emulsion was white and homogeneous in appearance. A light microscopy analysis did not show presence of active ingredient's crystals.

### EXAMPLE 9

A jellified oil in water emulsion having the following w/w % composition was prepared:

| | | |
|---|---|---|
| 1) | Metronidazole | 1.00% |
| 2) | Polycarbophil¹ | 1.00% |
| 3) | Glycerol USP | 15.00% |
| 4) | Peanut Oil | 9.00% |
| 5) | Acrylates/C10-C30 Alkyl Acrylates² | 1.00% |
| 6) | PEG-8 Caprylic/Capric Glyceride³ | 2.00% |
| 7) | Propylene Glycol USP | 25.00% |
| 8) | 2-Octyl Dodecanol⁴ | 11.00% |
| 9) | Benzyl Alcohol | 1.00% |
| 10) | Purified Water | 34.00% |

| | | |
|---|---|---|
| ¹Noveon AA1^{®}; ²Pemulen TR-1; ³Labrasol; ⁴Eutanol G | | |

The formulation was prepared by using the same method described for Example 1. The obtained jellified emulsion was white and homogeneous in appearance. A light microscopy analysis did not show presence of active ingredient's crystals.

### EXAMPLE 10

A jellified oil in water emulsion having the following w/w % composition was prepared:

| | | |
|---|---|---|
| 1) | Clotrimazole | 1.00% |
| 2) | Polycarbophil¹ | 1.00% |
| 3) | Glycerol USP | 15.00% |
| 4) | Peanut Oil | 9.00% |
| 5) | Acrylates/C10-C30 Alkyl Acrylates² | 1.00% |
| 6) | PEG-8 Caprylic/Capric Glyceride³ | 2.00% |
| 7) | Propylene Glycol USP | 25.00% |
| 8) | 2-Octyl Dodecanol⁴ | 11.00% |
| 9) | Benzyl Alcohol | 1.00% |
| 10) | Purified Water | 34.00% |

| | | |
|---|---|---|
| ¹Noveon AA1^{®}; ²Pemulen TR-1; ³Labrasol; ⁴Eutanol G | | |

The formulation was prepared by using the same method described for Example 1. The obtained jellified emulsion was white and homogeneous in appearance. A light microscopy analysis did not show presence of active ingredient's crystals.

### EXAMPLE 11

### Microscopy analysis

Active ingredient solubilisation and absence of crystals after storage was investigated by optical microscopy analysis.

The compositions employed were as follows: Example 1, 3, 5 and 9 compared to a commercial oil in water cream.

Pictures was taken by using a 20x objective lenses by carefully putting a small quantity of product on a glass slide.

A software of image analysis was used to compared different pictures. Pictures of same samples were taken also after 3 and 6 months of storage to evaluate the re-crystallisation of active ingredient.

It was concluded that Example 1, 3, 5 and 9 contain the active ingredient completely dissolved and it does not re-crystallise during storage at different temperatures, even for 6 months at 40°C.

### EXAMPLE 12

### Rheological evaluation

Microstructure properties of formulations have been evaluated by performing:
- **Rheological flow tests:** evaluation of flow curve (flow viscosity) and tixotropy;
- **Dynamic mechanical tests:** evaluation of small periodic deformations into the formulation, which determine breakdown or re-arrangement of structure; in the latter case, dynamic mechanical "strain sweep" test evaluated, under increased strain, the storage modulus G'(indicator of elastic behaviour) and the loss modulus G'' (measure of the dynamic viscous behaviour). Dynamic viscosity η' has been studied too as rate of energy dissipation in a viscoelastic material.

### Rheological flow tests

The compositions employed were as follows: Example 1, 4, 5 and 7 compared to a commercial oil in water cream.

Flow curves have been determined by using a Rheostress 600 rheometer, equipped with a cone/plate system (Ø= 35mm, angle= 2°), and Peltier temperature control, as below described:
step 1 Controlled-Shear (CS) mode 0.000 Pa - 1000. Pa 180.00 s
step 2 Controlled-Rate (CR) mode 0.000 1/s - 250.0 1/s 120.00 s
step 3 CR mode 250 1/s 30.00 s
step 4 CR mode 250 1/s - 0.000 1/s 120.00 s

Samples were applied to the lower plate by using a plastic spatula to ensure that the formulation shearing did not occur.

The whole set of measurement have been performed at least in triplicate at a constant temperature of 25'"G.

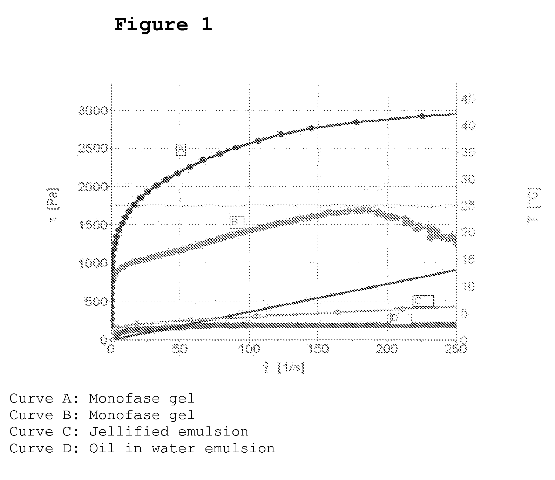

Results are shown in figure 1.

### Dynamic mechanical tests

The compositions employed were as follows: Example 1, 4 and 5 compared to a commercial oil in water cream Oscillation stress sweep and Frequency sweep measurements have been performed by using a Rheostress 600 rheometer, equipped with a cone/plate system (Ø= 35mm, angle= 2°), and Peltier temperature control as below described:
- Amplitude sweep study: sample was exposed to an increasing stress, from 0.000 Pa to 1000 Pa, at a constant frequency of 1 Hz. This test allowed the determination of the linear viscoelastic regime of the sample, and therefore the consequent choice of the stress value to use in the other oscillation tests.
- Frequency sweep study: perform in a CS mode, from 100 to 0.1 Hz, at a shear stress selected from the results of amplitude sweep (G' linear region).

All measurements, have been performed at least in triplicate on each test item and at a constant temperature of 25°C.

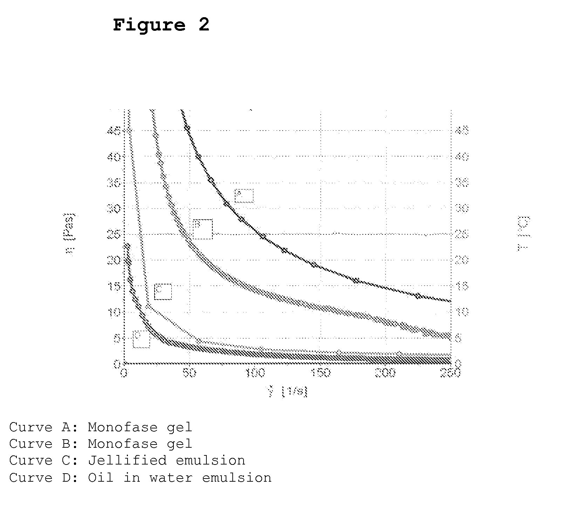

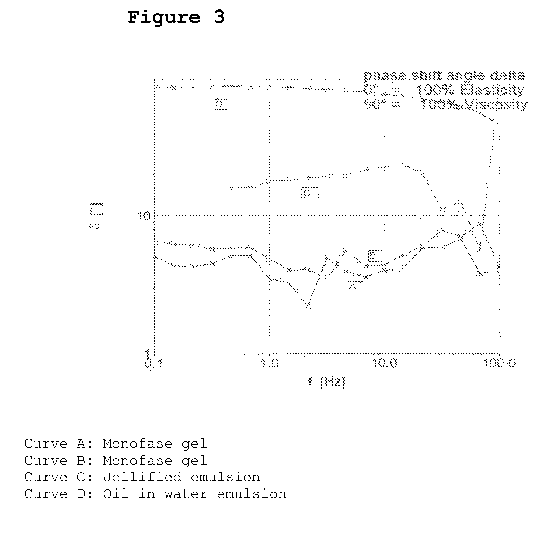

Results are shown in figures 2 and 3, respectively.

### EXAMPLE 13

### Release profile

The compositions employed were as follows: Example 1 and 7 compared to a commercial gel.

API's release has been evaluated by using the USP XXIV dissolution apparatus 2 equipped with the Enhancer Cell. Formulation was exactly weighted and packed into the Enhancer Cell. Therefore, only the upper surface of the semisolid was in contact with the dissolution medium phosphate buffer pH 4.5 separated by a GHP disk membrane (pore size: 0.45µm).

The Enhancer Cell was settled at the bottom of the vessels containing 500ml of the dissolution medium at a temperature of 37°C. The distance between the cell surface and the stirring paddle (50rpm) was 2cm. UV analysis was carried out every 5minutes at λ=305nm for a total time of 10hrs.

The results show that the release of active from formulations object of this inventions is slower than that from a common gel or cream.

## Claims

1. An oil in water emulsion containing (i) an internal hydrophobic phase in amounts from 1 to 40% by weight, (ii) an external hydrophilic phase in amounts from 60 to 99% by weight, (iii) at least a polymeric surfactant in amounts from 0.50 to 2.50% by weight and (iv) at least a water insoluble active principle in amounts from 0.01 to 25% by weight.

2. An emulsion according to claim 1, **characterized in that** said internal hydrophobic phase is present in amounts from 5 to 30% by weight, preferably from 10 to 25% by weight.

3. An emulsion according to claim 1, **characterized in that** said internal hydrophobic phase contains benzyl alcohol and 2-octyldodecanol.

4. An emulsion according to claim 1, **characterized in that** the weight ratio benzyl alcohol:2-octyldodecanol ranges from 1:3 to 1:13, preferably from 1:5 to 1:11.

5. An emulsion according to claim 1, **characterized in that** said external hydrophilic phase is present in amounts from 70 to 95% by weight, preferably from 75 to 90% by weight.

6. An emulsion according to claim 1, **characterized in that** said external hydrophilic phase contains water in amounts of up to 60% by weight.

7. An emulsion according to claim 1, **characterized in that** said external hydrophilic phase contains lower alkanols, polyhydric alcohols, polyethylene glycols, polypropylene glycols or mixtures thereof.

8. An emulsion according to claim 1, **characterized in that** said polymeric surfactant is present in amounts from 1.00 to.2.00% by weight.

9. An emulsion according to claim 1, **characterized in that** said polymeric surfactant is selected from the group of acrylates/C₁₀-C₃₀ alkyl acrilate crosspolymers and/or cellulose ethers.

10. An emulsion according to claim 9, **characterized in that** said cellulose ether is selected from alkylcellulose, preferably methylcellulose, and hydroxyalkylcellulose, preferably hydroxypropylmethylcellulose.

11. An emulsion according to claim 1, **characterized by** not containing neutralising agents.

12. An emulsion according to claim 1, **characterized by** containing a bio/mucoadhesive ingredient, preferably a Carbomer, in amounts from 0.5 to 1.5% by weight.

13. An emulsion according to claim 1, **characterized in that** said water insoluble active principle is present in amounts from 0.5 to 15% by weight, preferably from 1.0 to 10% by weight.

14. An emulsion according to claim 1, **characterized in that** said water insoluble active principle is selected from anti-bacterial drugs, anti-fungal drugs, anti-protozoal drugs, anti-inflammatory drugs and hormones.

15. An emulsion according to claim 1, **characterized in that** said water insoluble active principle is selected from Ciclopirox, Nifuratel, Estradiol, Imiquimod, Acyclovir, Metronidazole and Clotrimazole.

16. An emulsion according to claim 1, **characterized by** being a lotion, cream or gel.

17. An emulsion according to any of the preceeding claims, for topical application to the skin or into the vaginal cavity.
